# EUROPEAN PATENT SPECIFICATION

(11) **EP 0 920 495 B1**
(45) Date of publication and mention of the grant of the patent: **07.05.2008**
(21) Application number: 97934526.1
(22) Date of filing: 23.07.1997
(51) Int. Cl.: C12N 15/00, A01K 67/027, C07K 14/47, C12N 15/12, A61K 49/00

(54) **TRANSGENIC ANIMAL MODEL FOR ALZHEIMER DISEASE**
TRANSGENTIER-MODELL FÜR DIE KRANKHEIT VON ALZHEIMER
MODELE ANIMAL TRANSGENIQUE POUR LA MALADIE D'ALZHEIMER

(30) Priority: 24.07.1996 GB 9615569; 02.06.1997 GB 9711262
(43) Date of publication of application: 09.06.1999
(73) Proprietor: Novartis AG, 4056 Basel (CH); Novartis Pharma GmbH, 1230 Wien (AT)
(72) Inventor: SOMMER, Bernd, D-79591 Eimeldingen (DE); STAUFENBIEL, Matthias, D-79541 Haagen (DE)
(74) Representative: de Weerd, Petrus G.W.
(86) International application number: PCT/EP1997/003991
(87) International publication number: WO 1998/003644

(56) References cited:
- WO-A-93/14200
- WO-A-94/12627
- WO-A-95/11968
- GAMES D ET AL: "ALZHEIMER-TYPE NEUROPATHOLOGY IN TRANSGENIC MICE OVEREXPRESSING V717F BETA-AMYLOID PRECURSOR PROTEIN" NATURE, vol. 373, no. 6514, 9 February 1995, pages 523-527, XP000602050
- ANDRA, K. ET AL.: "Expression of APP in transgenic mice : a comparison of neuron-specific promoters" NEUROBIOLOGY OF AGING, vol. 17, no. 2, 23 May 1996, pages 183-190, XP002049072
- SOMMER, B. ET AL.: "Animal models for Alzheimer's disease based on genetic and pathology" SOCIETY FOR NEUROSCIENCE ABSTRACTS, vol. 22, no. 1-3, 16 - 21 November 1996, page 25 XP002049073

## Description

The present invention relates to an animal model useful for testing potential therapeutic agents for the treatment of neurodegenerative disorders, in particular Alzheimer's disease (AD).

More particularly the invention relates to an animal model involving transgenic manipulation of amyloid precursor protein (APP).

The lack of an experimental animal model for AD that reflects the pathological mechanisms is a major obstacle for both basic research and drug development. As one approach to such models, reproduction of characteristic lesions such as senile plaques, neurofibrillary pathology, and cell loss in certain areas of hippocampus and cortex can be attempted. However, it is presently unclear whether these lesions are cause or consequence of the disease process. An alternative approach for model generation is to use factors known to lead to the disease. Recently, genetic studies revealed mutations in APP, which cosegregate with early onset of familial AD in the fifth or sixth decade of life and follow an autosomal dominant inheritance pattern. Three distinct missense mutations affect codon 717 of APP (altering V717→I {hereinafter referred to as the London mutation}, V717→G and V717→F in the polypeptide), while codons 670/671 (altering K670→N and M671→L in the polypeptide, hereinafter referred to as the Swedish mutation) are altered in the APP gene of a Swedish AD pedigree (numbers according to APP770). These mutations flank the part of APP that gives rise to βA4, the principal component of the filaments deposited in plaques in the brains of AD patients. In vitro studies have indicated that the Swedish mutation leads to increased formation of a soluble form of βA4, while the APP717 mutations gives rise to a higher proportion of a longer βA4 variant which facilitates filament formation. Together with the finding that filamentous βA4 is toxic in vitro, this suggests that the APP mutations may lead to AD via a mechanism involving βA4, but other mechanisms cannot be excluded.

More recently, transgenic mice have been generated, expressing APP with mutations in codons 717 and 670/671, using several neuron-specific promotors to drive expression of human APP cDNAs. Although protein levels reaching or exceeding the amount of endogenous APP have been obtained, the full pattern of histological alterations characteristic of AD have not been seen in the transgenic mice.

It has now surprisingly been found that by appropriate selection of APP expression construct, high levels of transgene mRNA are obtained, which exceed the endogenous APP message by up to 10 fold, and result in correspondingly elevated protein levels. Moreover, on histological analysis, significant deposits of human βA4 peptide are observed. Additionally and even more importantly, hyperphosphorylation of the microtubule-associated protein tau is achieved, which is a pathological phenotype associated to AD. Furthermore, the deposits accumulate cholinesterase staining associated with a local distorsion of cholinergic fibers typically observed in AD. Both features have not been reported previously with analogous transgenic animals. The pathology is accompanied with selective neuron loss in distinct areas of the brain.

Accordingly in a first aspect the invention provides a recombinant DNA construct comprising a polynucleotide encoding a human APP polypeptide comprising the Swedish mutation, functionally linked to a Thy-1 promoter element, characterized in that the Thy-1 promoter element is a rodent, e.g. mouse, Thy-1 promoter element and the Swedish mutation is the only mutation present in the APP polypeptide.

Transgenic mice expressing said mutated human APP under control of said promotor have been found to develop a pathological phenotype which goes beyond that previously described by Games et al. [Nature 373, 523-527 (1995)], by combining APP and tau linked features of the AD pathology. Moreover, the mice have been found to present behavioural changes characteristic of AD, which has also never been reported before with transgenic animals.

It will be appreciated that such mice, by closely reflecting the AD pathology, as well as their transgenic cells, are particularly useful models of the disease.

Accordingly in a further aspect the invention provides transgenic mice which exhibit both APP and tau-linked features, e.g. histological features, of AD pathology, and preferably also behavioural changes characteristic of AD.

The transgenic mice express a human APP comprising the Swedish mutation. Suitably also the transgenic mouse exhibits the features of AD pathology before 12 months of age preferably by about 6 months of age. This aspect of the invention includes transgenic cells derived from the transgenic mouse.

Without prejudice to the generality of the present invention, it appears that the level at which the transgene is expressed in the transgenic animal e.g. the level of transgene mRNA, is an important factor for obtaining AD pathology in the animal.

Also disclosed is transgenic mouse cell, wherein DNA coding for a human APP having only the swedish mutation is expressed at such a level that the amount of transgene mRNA exceeds the endogenous APP message by about 5 times, e.g. from 3 to 6 times, or more, e.g. from about 5 to about 10 times, as well as a transgenic mouse, in the cells of which DNA coding for a human APP having only the Swedish mutation is expressed at such a level that the amount of transgene mRNA exceeds the endogenous message by about 5 times or more.

It furthermore appears that the number of genetic lesions influencing the production of βA4 introduced in a transgenic animal is another important factor for obtaining AD pathology in the animal.

The DNA coding for human APP may comprise cDNA and/or genomic DNA, and is conveniently cDNA.

More particularly the present invention provides a transgenic mouse cell, wherein DNA encoding a human APP polypeptide comprising the Swedish mutation is expressed under the transcriptional control of a rodent Thy-1 promotor element, as well as a transgenic mouse, in the cells of which DNA encoding a human APP polypeptide comprising the Swedish mutation is expressed under the transcriptional control of a Thy-1 promotor element, characterized in that the Swedish mutation is the only mutation present in the APP polypeptide and the Thy-1 promoter element is a rodent, e.g. mouse, Thy-1 promoter element.

Transgenic mice according to the invention include mice into which the construct has been introduced directly as well as progeny of such mice which retain the ability to express the construct.

Cells manipulated according to the invention may be prepared by any known transfection technique. The DNA sequence may be introduced by direct genetic manipulation or into an earlier generation of the cell. Thus, the cells may be obtained from transgenic animals and cultured *in vitro.*

Also the transgenic mice may be generated according to well established methods, such as manipulation of embryos, e.g. by gene transfer into embryonic stem cells, retroviral infection of early embryos or pronuclear microinjection.

The pronuclear microinjection technique is preferred. Transcription units obtained from a recombinant DNA construct of the invention are injected into pronuclei of mouse embryos and the obtained founder transgenics are bred.

The results obtained in the offspring can be analysed using various techniques well known in the art. Thus, for example, transgene APP mRNA expression is analysed by RNA blotting, the expression pattern of the transgene in the brain is determined by *in situ* hybridization, detection of APP in the brain is effected using immunoblotting techniques (western blot analysis) and the effects of the expression are studied by histology and immunohistology.

Models based on cells and animals of the invention may be used for example to identify and assess the efficacy of potential therapeutic agents in neurodegenerative diseases, particularly in diseases where βA4 peptide is deposited and/or the microtubule-associated protein tau is hyperphosphorylated, more particularly in AD. In particular such models may be used in screening or characterization assays for detecting agents likely to prevent βA4 deposit and/or hyperphosphorylation of tau.

Accordingly in a further aspect the invention comprises a method for testing a potential therapeutic agent for the treatment of Alzheimer's disease, wherein a cell of the invention is used as target cell. More particularly it comprises such a method, wherein the agent is administered to a transgenic mouse of the invention. Moreover the invention comprises a screening or characterization assay consisting in or including such a method, as well as a screening assay kit comprising cells of the invention.

Methods for screening potential therapeutic agents using cell lines or animals are well known in the art. The cells and animals of the present invention may be used in analogous manner.

The recombinant cells may for example be incubated with the potential therapeutic agent and with antibodies recognizing βA4 amyloid in typical senile and diffuse plaques and/or with tau antibodies staining neurofibrillary tangles in the Alzheimer brain. In methods where the transgenic mice themselves are used, the effects of the potential therapeutic agent may be determined by carrying out various investigations on the animals after sacrifice. Also after administration of the potential therapeutic agent, the transgenic mouse may undergo behavioural testing in order to monitor cognitive function.

The techniques of detection of βA4 and protein tau, including Western blot analysis, and the antibodies used therefor, are also well documented.

Compounds for use in the treatment of neurodegenerative diseases, which have been identified using an assay or assay kit as defined above, are also part of the present invention.

### The following example illustrates the invention:

### Expression construct

Human APP751 cDNA carrying the Swedish double mutation is modified at the 5' end to reconstitute an optimal translation initiation sequence (GCC GCC ATG G).

This cDNA starting at above sequence and extending to nucleotide 3026 (Hind III site) is inserted into the Xho I cloning site of a pUC18-based vector containing an 8.1 kb EcoRI fragment comprising the mouse Thy-1.2 gene [Vidal et al. (1990) EMBO J. 9, 833-840]. The vector is modified such that a 1.5kb BanI-Xhol fragment carrying exon 3 and flanking intervening sequences is replaced by a linker sequence encoding the unique Xho I recognition site [Moechars et al. (1996) EMBO J. 15, 1265-1274]. Transcription units are released by Notl/Pvul digestion.
Expression construct APP 14 described in K. Andrä et al., Neurobiology of Aging, Vol. 17, No. 2, 183-190 (1996) is modified by replacing a 600 bp Bgl II/Spe I fragment with a corresponding fragment of a human APP₇₅₁ cDNA carrying the London mutation V 717 → I. Transcription units are released by Not I digestion.

### Generation of transgenic mice

Isolated transcription units are injected into the pronuclei of B6D2F1 x B6D2F1 embryos to generate transgenic founder animals.

**Northern blot analysis, in situ hybridization, western blot analysis, histology and immunohistology**
are performed according to the methods described in K. Andrä et al., Neurobiology of Aging, Vol. 17, No. 2,183-190 (1996).

### Results

Offspring of the founder animals express human APP mRNA in high amounts throughout all brain structures as demonstrated by *in situ* hybridization. Determined amounts of transgene derived protein exceed those of endogenous APP 5 to 10 fold. At 6 months of age, these mice show extracellular deposits of human βA4 peptide in cerebral cortex and the hippocampal formation. These deposits are positive in methenamine silver impregnation, thioflavin S staining and in Congo Red birefringence. They are surrounded by reactive astrocytes and dystrophic neurites. In addition, plaques are immunoreactive with antisera specific to hyperphosphorylated microtubule associated protein tau as found in brains of AD patients, which has not been reported previously for analogous transgenic animals. Hence, the described deposits in the brains of these mice closely resemble senile plaques found in AD patients. When stained for acetylcholinesterase, a strong labelling of plaques and a local distorsion of the cholinergic fibre network is observed. Plaques contain acetylcholinesterase activity in structures resembling swollen, dystrophic neurites. This degeneration of cholinergic neurites is another well-known feature associated with AD. Furthermore, a local degeneration of neurons in the plaque vicinity is observed in areas typically affected in AD such as hippocampal CA1. Here, the neuron loss is negatively correlated to the plaque burden and can reach up to 20%.

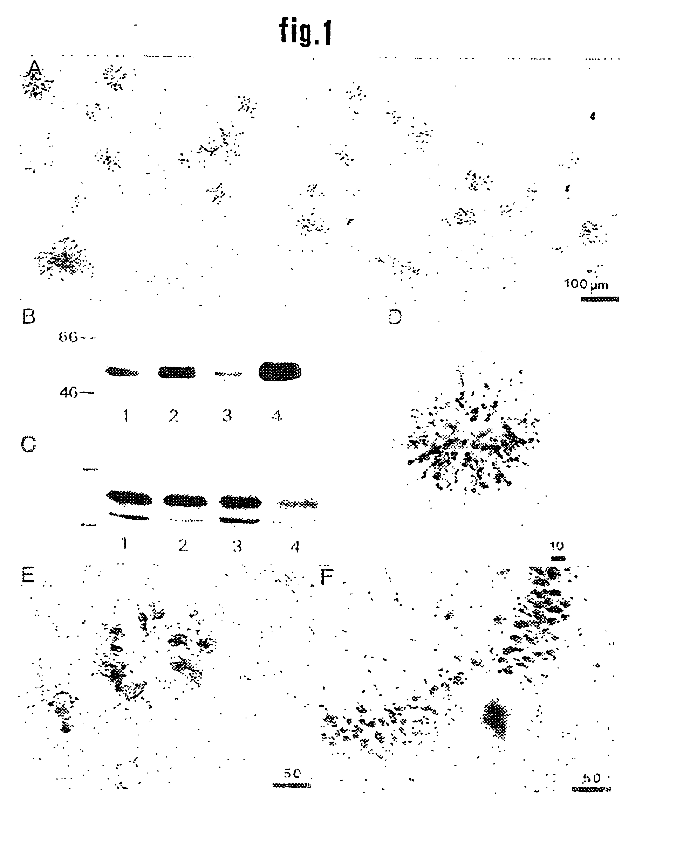

Tau hyperphosphorylation, cholinesterase staining and neuron loss in APP transgenic mice according to the invention are illustrated in Figure 1. Staining of plaques with tau antibody AT8 recognizing phosphorylated Ser202 and Thr205 of tau is shown on a sagital free floating section of a transgenic mouse brain in A and in higher magnification in D. Western blots of brain extracts from transgenic mice, 6 months (2) and 15 months (4) of age and littermate controls (1,3) are shown in B and C. Blots were stained with antibodies AT8 (B) and N-tau7 (C) recognizing tau in a phosphorylation dependent and independent manner, respectively. Numbers indicate molecular weights of marker proteins in kDa. E shows staining for acetylcholine esterase in transgenic mice. A local distorsion of cholinergic fibers in the plaque vicinity can be noted. The loss of pyramidal neurons in the vicinity of Aβ deposits in area CA3 is shown in F by toluidine blue staining.

### Behavioural testing

Transgenic mice obtained as described above show significant non-cognitive behavioural changes corresponding to changes observed with patients suffering from AD, as reported by Mega et al. (1996) Neurology 46, 130-135.

For example in the Half-Enclosed Platform test according to a modification of Käsermann (1986) Psychopharmacol. 89, 31-37, compared to non-transgenic littermates, the animals avoided the open half and an increase of exploratory-behavioural moves and postures such as locomotion and head raising, indicative of agitation, disinhibition and irritability as reported for AD patients was observed.

### Cognitive testing

Furthermore the mice show significant cognitive impairment.

For example in the water maze according to Morris et al. (1982) Nature 297, 681-683, compared to non-transgenic littermates, the animals made significantly less crossings of the annulus representing the platform's previous position (2.5 ± 0.5 vs. 4.4 ± 0.7; p < 0.05, 2-tail Mann-Whitney U-test) and spent a significantly lower percentage of time in the quadrant containing the annulus (20.8 ± 3.8 vs. 33.1 ± 3.2; p < 0.05, 2-tail Mann-Whitney U-test).

## Claims

1. A recombinant DNA construct comprising a polynucleotide encoding a human APP polypeptide comprising the Swedish mutation, functionally linked to a Thy-1 promoter element, **characterized in that** the said Thy-1 promoter element is a rodent Thy-1 promoter element and the said Swedish mutation is the only mutation present in the said APP polypeptide.

2. A transgenic mouse cell, wherein DNA encoding a human APP polypeptide comprising the Swedish mutation is expressed under the transcriptional control of a Thy-1 promoter element, **characterized in that** the said Swedish mutation is the only mutation present in the said APP polypeptide and the said Thy-1 promoter element is a rodent, e. g. mouse, Thy-1 promoter element.

3. A transgenic mouse, **characterized in that** it expresses a recombinant DNA construct as claimed in claim 1 and exhibits APP and tau-linked features of the AD pathology.

4. A transgenic mouse according to claim 3, in the cells of which mouse DNA encoding a human APP polypeptide comprising the Swedish mutation is expressed under the transcriptional control of a Thy-1 promoter element, **characterized in that** the said Swedish mutation is the only mutation present in the said APP polypeptide and the said Thy-1 promoter element is a rodent Thy-1 promoter element.

5. A method of generating a transgenic mouse as defined in claim 3, **characterized in that** the said mouse is generated by incorporating a recombinant DNA construct as claimed in claim 1 into the genome of a mouse.

6. A method according to claim 5, **characterized in that** it comprises the injection of transcription units obtained from a recombinant DNA construct as claimed in claim 1 into a pronucleus of a mouse embryo and the breeding of the so obtained founder mouse.

7. A method of testing a potential therapeutic agent for the treatment of Alzheimer's disease, **characterized in that** either a transgenic mouse as claimed in claim 3 or claim 4 is used as animal model or a cell as claimed in claim 2 is used as target cell.

8. A method according to claim 7, **characterized in that** the potential therapeutic agent is administered to a transgenic mouse generated by a method as claimed in claim 5 or claim 6.

9. A method according to claim 7, **characterized in that** the potential therapeutic agent is administered to a transgenic mouse as claimed in claim 3 or claim 4, or is contacted with a cell as claimed in claim 2, and the βA4 deposit and/or the hyperphosphorylation of tau is/are determined.

10. A screening or characterization assay, **characterized in that** it consists of, or includes, a method as claimed in any one of claims 7 to 9.

11. A screening assay kit, **characterized in that** it comprises a cell as claimed in claim 2.

## Patentansprüche

1. Rekombinantes DNA-Konstrukt, umfassend ein Polynucleotid, das für ein humanes APP-Polypeptid kodiert, das die Schwedische Mutation umfasst, die funktional an ein Thy-1 Promotorelement gebunden ist, **dadurch gekennzeichnet, dass** das Thy-1 Promotorelement ein Nager Thy-1 Promotorelement ist und die Schwedische Mutation die einzige Mutation im APP-Polypeptid ist.

2. Transgene Mauszelle, worin die für ein humanes APP-Polypeptid kodierende DNA, die die Schwedische Mutation umfasst, unter der transkriptionalen Kontrolle eines Thy-1 Promotorelements exprimiert wird, **dadurch gekennzeichnet, dass** die Schwedische Mutation die einzige Mutation im APP-Polypeptid ist und das Thy-1 Promotorelement ein Nager Thy-1 Promotorelement ist, beispielsweise von einer Maus.

3. Transgene Maus, **dadurch gekennzeichnet, dass** diese ein rekombinantes DNA-Konstrukt wie im Anspruch 1 exprimiert und APP und tau-verknüpfte Merkmale der AD Pathologie exhibiert.

4. Transgene Maus nach Anspruch 3, wobei in den Zellen dieser Maus eine DNA, die für ein humanes APP-Polypeptid kodiert, das die Schwedische Mutation umfasst, exprimiert wird unter der transkriptionalen Kontrolle eines Thy-1 Promotorelements, **dadurch gekennzeichnet, dass** diese Schwedische Mutation die einzige Mutation ist, die im APP-Polypeptid vorhanden ist und das Thy-1 Promotorelement ein Nager Thy-1 Promotorelement ist.

5. Verfahren zur Erzeugung einer transgenen Maus gemäß Definition im Anspruch 1, **dadurch gekennzeichnet, dass** diese Maus generiert wird durch Inkorporation eines rekombinanten DNA-Konstrukts gemäß Anspruch 1 in das Genom einer Maus.

6. Verfahren nach Anspruch 5, **dadurch gekennzeichnet, dass** dieses umfasst die Injektion von Transkriptionseinheiten, die von einem rekombinanten DNA-Konstrukt gemäß Anspruch 1 erhalten worden sind, in einen Pronucleus eines Mausembryos und durch Brütung der so erhaltenen Stiftermaus.

7. Verfahren zur Testung eines potentiellen therapeutischen Mittels für die Behandlung von Alzheimer-Krankheit, **dadurch gekennzeichnet, dass** als Tiermodell entweder eine transgene Maus nach Anspruch 3 oder 4 verwendet wird, oder als Zielzelle eine Zelle nach Anspruch 2 angewandt wird.

8. Verfahren nach Anspruch 7, **dadurch gekennzeichnet, dass** das potentielle therapeutische Mittel einer transgenen Maus verabreicht wird, die nach einem Verfahren von Anspruch 5 oder 6 generiert worden ist.

9. Verfahren nach Anspruch 7, **dadurch gekennzeichnet, dass** das potentielle therapeutische Mittel einer transgenen Maus nach Anspruch 3 oder 4 verabreicht wird oder mit einer Zelle nach Anspruch 2 kontaktiert wird, und das βA4 Depot und/oder die Hyperphosphorylierung von tau bestimmt wird.

10. Screeningassay oder Charakterisierungsassay, **dadurch gekennzeichnet, dass** dieser besteht aus einem oder beinhaltet ein Verfahren nach einem der Ansprüche 7 bis 9.

11. Screeningassaybausatz, **dadurch gekennzeichnet, dass** dieser eine Zelle nach Anspruch 2 umfasst.

## Revendications

1. Construction d'ADN recombinant comprenant un polynucléotide codant pour un polypeptide APP humain comprenant la mutation suédoise, lié de manière fonctionnelle à un élément promoteur Thy-1, **caractérisée en ce que** ledit élément promoteur Thy-1 est un élément promoteur Thy-1 de rongeur et ladite mutation suédoise est la seule mutation présente dans ledit polypeptide APP.

2. Cellule de souris transgénique, dans laquelle de l'ADN codant pour un polypeptide APP humain comprenant la mutation suédoise est exprimé sous le contrôle transcriptionnel d'un élément promoteur Thy-1, **caractérisée en ce que** ladite mutation suédoise est la seule mutation présente dans ledit polypeptide APP et ledit élément promoteur Thy-1 est un élément promoteur Thy-1 de rongeur, par exemple de souris.

3. Souris transgénique, **caractérisée en ce qu'**elle exprime une construction d'ADN recombinant selon la revendication 1 et présente les caractéristiques liées à APP et tau de la pathologie d'Alzheimer.

4. Souris transgénique selon la revendication 3, dans les cellules de laquelle de l'ADN codant pour un polypeptide APP humain comprenant la mutation suédoise est exprimé sous le contrôle transcriptionnel d'un élément promoteur Thy-1, **caractérisée en ce que** ladite mutation suédoise est la seule mutation présente dans ledit polypeptide APP et ledit élément promoteur Thy-1 est un élément promoteur Thy-1 de rongeur.

5. Procédé de génération d'une souris transgénique telle que définie dans la revendication 3, **caractérisé en ce que** ladite souris est engendrée en incorporant une construction d'ADN recombinant selon la revendication 1 dans le génome d'une souris.

6. Procédé selon la revendication 5, **caractérisé en ce qu'**il comprend l'injection d'unités de transcription obtenues à partir d'une construction d'ADN recombinant selon la revendication 1 dans un pronoyau d'un embryon de souris et l'élevage de la souris fondatrice ainsi obtenue.

7. Procédé pour tester un agent thérapeutique potentiel pour le traitement de la maladie d'Alzheimer, **caractérisé en ce que** soit une souris transgénique selon la revendication 3 ou la revendication 4 est utilisée comme modèle animal, soit une cellule selon la revendication 2 est utilisée comme cellule cible.

8. Procédé selon la revendication 7, **caractérisé en ce que** l'agent thérapeutique potentiel est administré à une souris transgénique engendrée par un procédé selon la revendication 5 ou la revendication 6.

9. Procédé selon la revendication 7, **caractérisé en ce que** l'agent thérapeutique potentiel est administré à une souris transgénique selon la revendication 3 ou la revendication 4, ou est mis en contact avec une cellule selon la revendication 2, et le dépôt de βA4 et/ou l'hyperphosphorylation de tau est/sont déterminé(s).

10. Test de criblage ou de caractérisation, **caractérisé en ce qu'**il consiste en, ou comprend, un procédé selon l'une quelconque des revendications 7 à 9.

11. Trousse de test de criblage, **caractérisée en ce qu'**elle comprend une cellule selon la revendication 2.
